# EUROPEAN PATENT SPECIFICATION

(11) **EP 2 160 184 B1**
(45) Date of publication and mention of the grant of the patent: **07.03.2018**
(21) Application number: 08754476.3
(22) Date of filing: 14.05.2008
(51) Int. Cl.: A61K 9/26, A61K 9/22, A61K 31/58, A61M 31/00, A61K 9/00, A61K 9/16, A61K 9/19

(54) **HYPERCOMPRESSED PARTICLES FOR CONTROLLED RELEASE OF OPHTHALMIC MEDICATIONS**
HYPERKOMPRIMIERTE TEILCHEN ZUR KONTROLLIERTEN FREISETZUNG VON OPHTHALMISCHEN ARZNEIMITTELN
PARTICULES HYPERCOMPRIMÉES POUR LIBÉRATION CONTRÔLÉE DE MÉDICAMENTS OPHTALMIQUES

(30) Priority: 14.05.2007 US 930105 P
(43) Date of publication of application: 10.03.2010
(73) Proprietor: Sustained Nano Systems Llc, Great Neck, NY 11023 (US)
(72) Inventor: LIBIN, Barry, Great Neck, NY 11023 (US); LIEBMANN, Jeffrey M., Great Neck, NY 11023 (US); CHEN, Weiliam, MT Sinai, NY 11766 (US)
(74) Representative: Bonatto, Marco
(86) International application number: PCT/US2008/006195
(87) International publication number: WO 2008/143906

(56) References cited:
- EP-A1- 1 477 187
- EP-A2- 0 251 680
- WO-A1-99/48519
- WO-A1-2006/028361
- JP-A- 05 065 221
- US-A- 4 478 818
- US-A- 5 501 856
- US-A1- 2002 111 603

## Description

### FIELD OF THE INVENTION

This invention relates to the field of controlled release ophthalmic dispensing devices.

### BACKGROUND OF THE INVENTION

There is a widespread recognition in the field of ophthalmology that controlled release drug delivery systems would benefit patient care and ocular health by providing extended delivery of therapeutic agents to the eye while minimizing the problems associated with patient compliance to prescribed therapeutic medical regimens. Although a wide variety of drug delivery methods exist, topical eye drop therapy is limited by poor absorption, a need for frequent and/or chronic dosing over periods of days to years, rapid turnover of aqueous humor, production and movement of the tear film and other causes, which may effectively remove therapeutic agents long before therapy has been completed or the proper dose delivered.
Two sustained delivery systems in the form of ophthalmic inserts that have been developed for commercial use are the Ocusert system (Akorn) and Lacrisert® (Aton). The Ocusert device is designed to provide for the release of medication at predetermined and predictable rates, which permits the elimination of frequent dosing by the patient, ensures nighttime medication, and provides a better means of patient compliance. The insert is elliptical with dimensions of 13.4 by 4.7 mm and 0.3 mm in thickness. The insert is flexible and is a multilayered structure consisting of a drug-containing core surrounded on each side by a layer of copolymer membranes through which the drug diffuses at a constant rate. The rate of drug diffusion is controlled by the polymer composition, the membrane thickness, and the solubility of the drug. The devices are sterile and do not contain preservatives. Ocusert inserts containing pilocarpine have been used in glaucoma therapy. After placement in the conjunctival fornix, the inserts are designed to release medication at the desired rates over a 7-day period at which time they are removed and replaced with new ones.

The Lacrisert® insert is a sterile, translucent, rod-shaped, water-soluble form of hydroxypropyl cellulose. The product is inserted into the inferior cul-de-sac of the eye of patients with dry eye states. The insert acts to stabilize and thicken the precorneal tear film and to delay its breakup. Inserts are typically placed in the eye once or twice daily. Following administration, the inserts soften and slowly dissolve.

The following U.S. patents disclose various ocular inserts for medicinal therapy. U.S. 4,730,013 to J.V. Bondi, et al., assigned to Merck & Company, Inc., discloses ocular inserts with or without pharmaceutically active agents, comprising 75% to 100% of a matrix of 15% polyvinyl alcohol, 10% glycerine, 75% hydroxy propyl methylcellulose phthalate, and 0-25% of a pharmacologically active agent.

U.S. 4,522,829 to Andreas Fuchs, et al., (Merck GmbH), discloses an intraocular pressure-lowering film insert of a 1-(p-2-iso-propoxyethoxy methyl-phenoxy)-3-isopropylamino-propan-2-ol or a physiologically acceptable salt thereof and an ophthalinically acceptable carrier.

U.S. 4,432,964 to Robert M. Gale (Alza Corp.) discloses an ocular insert for treating inflammation made of a pair of micronized steroids consisting of two topically acceptable different chemical therapeutic forms of betamethasone or a derivative, and a bio-erodible polymeric polyorthoester carrier.

U.S. 4,346,709 to Edward E. Schmitt (Alza Corp.) discloses an erodible device for delivering a drug to an environment of use, which includes a poly(orthoester) or a poly(orthocarbonate).

U.S. 4,303,637 to Robert M. Gale, et al., discloses an ocular insert composed of a beta blocking drug in a polymer with the drug surrounded by the polymer selected from the group consisting of poly(olefin), poly(vinylolefin), poly(haloolefin), poly(styrene), poly(vinyl), poly(acrylate), poly(methacrylate), poly(oxide), poly(ester), poly(amide), and poly(carbonate).

U.S. 4,190,642 (Alza Corp.) discloses an ocular insert composed of a discrete depot of a pilocarpine solute and an epinephrine solute, a film of an ethylene-vinyl ester copolymer forming the insert, where fluid from the environment is imbibed through the wall into the depots to continually dissolve the solutes and release the formulation.

U.S. 4,093,709 to Nam S. Choi (Alza Corp.) discloses an ocular insert composed of an orthoester and an orthocarbonate polymer.

U.S. 3,993,071, issued Nov. 23, 1976 to Takeru Higuchi, et al., discloses a bio-erodible ocular insert for the controlled administration of a drug to the eye from 8 hours to 30 days, in which the drug formulation can also be microencapsulated and the microcapsules dispersed in the drug release rate controlling material.

U.S. 3,981,303 to Takeru Higuchi, et al. (Alza Corp.) discloses an ocular insert for the continuous controlled administration of a drug to the eye composed of a plurality of microcapsule reservoirs comprised of a drug formulation confined within a drug release rate controlling material, distributed throughout a bio-erodible matrix permeable to the passage of the drug at a higher rate than the rate of drug passage through the drug release rate controlling material, where the device is of an initial shape and size that is adapted for insertion and retention in the sac of the eye. The hydrophobic material may be selected from cholesterol, waxes, C.sub.10 to C.sub.20 fatty acids, and polyesters, and the drug may be selected from epinephrine, pilocarpine, hydrocortisone, idoxuridine, tetracycline, polymixin, gentamycin, neomycin, and dexamethasone.

U.S. 3,960,150 to Takeru Higuchi, et al. (Alza Corp.) discloses an ocular insert for the continuous controlled administration of a drug to the eye composed of a body of hydrophobic bio-erodible drug release rate controlling material containing a drug, where the body is of an initial shape adapted for insertion in the sac of the eye, where the drug release rate controlling material can be a polyester, and the drug may be selected from epinephrine, pilocarpine, hydrocortisone, idoxuridine, tetracycline, polymixin, gentamycin, neomycin, and dexamethasone, and derivatives.

U.S. 3,811,444, issued May 21, 1974 to Richard W. Baker, et al., assigned to the Alza Corp., discloses an ocular insert for the continuous controlled administration of a drug to the eye comprising a drug formulation dispersed through a body of selected hydrophobic polycarboxylic acid which erodes over time to dispense the desired amount of drug. The polycarboxylic acid can be a copolymer of an acid from the group of maleic acid, acrylic acid, lower alkyl acrylic acids from about 4 to about 6 carbon atoms, with a copolymerizable olefinically unsaturated material selected from the group consisting of ethylene, propylene, butadiene, isoprene and styrene and the lower alkyl vinyl ethers.

U.S. Pat. No. 3,630,200, issued Dec. 28, 1971, to Takeru Higuchi, assigned to the Alza Corporation, discloses a drug-dispensing ocular insert for insertion into the cul-de-sac of the conjunctiva between the sclera of the eyeball and the lid where the inner core contains the drug and is surrounded by a soft hydrophilic outer layer, where the outer layer can be composed of a polymer selected from the group consisting of hydrophilic hydrogel of an ester of acrylic or methacrylic acid, modified collagen, cross-linked hydrophilic polyether gel, cross-linked polyvinyl alcohol, and cross-linked partially hydrolyzed polyvinyl acetate and cellulosic gel. The inner core may be a polymer selected from the group of plasticized or unplasticized polyvinylchloride, plasticized nylon, unplasticized soft nylon, silicone rubber, polyethylene, hydrophilic hydrogel of an ester of acrylic or methacrylic acid, modified collagen, cross-linked hydrophilic polyether gel, cross-linked polyvinyl alcohol, cross-linked partially-hydrolyzed polyvinylacetate, cellulosic gel, ion-exchange resin and plasticized polyethylene terephthalate.

U.S. Pat. No. 3,618,604 to Richard A. Mess (Alza Corporation) discloses a drug-dispensing ocular insert adapted for insertion into the cul-de-sac of the eye, where the insert is a tablet containing a reservoir of drug formulation within a flexible polymeric material, and the polymeric material is formed of plasticized or unplasticized polyvinylchloride, plasticized nylon, unplasticized soft nylon, plasticized polyethylene terephthalate, silicon rubber, hydrophilic hydrogel of a ester of acrylic or methacrylic acid, modified collagen, cross-linked hydrophilic polyether gel, cross-linked polyvinyl alcohol, and cross-linked partially-hydrolyzed polyvinylacetate.

U.S. Pat. Nos. 3,993,071; 3,986,510; 3,981,303, 3,960,150, and 3,995,635 to Higuchi, et al., disclose a biodegradable ocular insert made from zinc alginate, poly(lactic acid), poly(vinyl alcohol), poly(anhydrides), and poly(glycolic acid).

A number of patents disclose the use of drug-loaded polyanhydrides (wherein the anhydride is in the backbone of the polymer) as matrix materials for ocular inserts. See, in general, U.S. 5,270,419; 5,240,963; and 5,137,728. Other U.S. patents that describe the use of polyanhydrides for controlled delivery of substances include: U.S. 4,857,311 to Domb and Langer, entitled "Polyanhydrides with Improved Hydrolytic Degradation Properties," which describes polyanhydrides with a uniform distribution of aliphatic and aromatic residues in the chain, prepared by polymerizing a dicarboxylic acid with an aromatic end and an aliphatic end); U.S. 4,888,176 to Langer, Domb, Laurencin, and Mathiowitz, entitled "Controlled Drug Delivery High Molecular Weight Polyanhydrides," which describes the preparation of high molecular weight polyanhydrides in combination with bioactive compounds for use in controlled delivery devices); and U.S. 4,789,724 to Domb and Langer, entitled "Preparation of Anhydride Copolymers," which describes the preparation of very pure anhydride copolymers of aromatic and aliphatic diacids.

U.S. 5,075,104 discloses an ophthalmic carboxyvinyl polymer gel for the treatment of dry eye syndrome.

U.S.. 4,407,792 discloses an aqueous gel that includes a gel-forming amount of an ethylene-maleic anhydride polymer.
U.S. 4,248,855 discloses the salt of pilocarpine with a polymer containing acid groups for use as an ocular insert, among other things.
U.S. 4,180,064 and U.S. 4,014,987 disclose the use of poly(carboxylic acids) or their partially esterified derivatives as drug delivery devices.
PCT/US90/07652 discloses that biologically active compounds containing a carboxylic acid group can be delivered in the form of an anhydride of a carrier molecule that modifies the properties of the molecule.

EP0251680 A2 discloses bioerodible compositions for controlled release of a drug to a body fluid comprising an external matrix rapidly soluble in the body fluid and bioerodible microparticles dispersed in the external matrix. The composition may be an ocular dosage form. The external matrix can comprise e.g. glycolic acid, lactic acid. These materials can be used in lyophilized form by mixing the microparticles with an amount of matrix material and preparing a dosage form by direct compression. The materials used to form the microparticles can be poly(glycolic acid), poly(lactic acid). Ophthalmic drugs, such as corticosteroids can be incorporated into the matrix material. Although these patents disclose a number of types of ocular inserts, there is still a need to provide new dosage forms with modified properties for the delivery of ophthalmic therapeutic agents. In particular, there is a need to provide an ophthalmic dispensing device that provides for the long acting delivery of ophthalmic therapeutic agents to the eye. The formulations comprise a matrix of a polymer carrier and an active drug where the matrix is made by compression of micro or nano particles of an ophthalmic therapeutic agent in combination with a polymer. The matrix is positioned in or near the eye where it will make available the ophthalmic agent for treating pathologic conditions in the eye. The preferred polymeric matrix combines the characteristics of stability, strength, flexibility, low melting point, dispersability and suitable degradation profile. The matrix must retain its integrity for a suitable time so that it may be handled and placed in an aqueous environment, such as the eye, without loss of structural integrity. It should also be stable enough to be stored an shipped without loss of structural integrity. The matrix is designed to disintegrate into its constituent particles shortly after it is placed in position to release the ophthalmic therapeutic agent.

### SUMMARY OF THE INVENTION

The invention provides a controlled release ophthalmic dispensing device that comprises a matrix that is made by hypercompressing units of microparticles or nanoparticles that comprise a therapeutically compatible polymer and an ophthalmic therapeutic agent, as defined in claim 1. The hypercompressed unit is shaped in such a manner that the unit may be positioned in the conjunctival fornix, subconjunctival, sub-Tenon's, episcleral, intrascleral, parabulbar, retrobulbar, or intraocular spaces without causing any substantial patient discomfort during the time that it is retained in the eye. Described herein is also a method of administering an ophthalmic therapeutic agent which comprises (a) forming a dosage form comprising a polymer in combination with an ophthalmic therapeutic agent in the form of microparticles or nanoparticles; (b) hypercompressing the microparticles or nanoparticles to form a controlled release ophthalmic dispensing unit; and (c) thereafter positioning said ophthalmic dispensing unit in the eye of a patient requiring the administration of said ophthalmic therapeutic agent.
Accordingly, it is an object of the invention to provide an ophthalmic dispensing device for use in the conjunctival fornix, subconjunctival, sub-Tenon's, episcleral, intrascleral, parabulbar, retrobulbar, or intraocular spaces that will release ophthalmic therapeutic agents over a period of time.
It is therefore an object of the present invention to provide an ophthalmic dispensing device that provides for the controlled release of ophthalmic therapeutic agents for the treatment of pathologic eye conditions.
It is also an object of the invention to provide an ophthalmic dispensing device that is made by hypercompressing microparticles or nanoparticles containing an ophthalmic therapeutic agent and a compressed polymer which will release the ophthalmic therapeutic agent over an extended period of time.
It is also an object of this invention to avoid active patient involvement with the administration of an ophthalmic therapeutic agent by having a physician place an ophthalmic dispensing device in a position where it will deliver the ophthalmic therapeutic agent to the eye over an extended period of time without any action on the part of the patient.

It is also an object of this invention to provide an ophthalmic dispensing device that will provide controlled release of an ophthalmic therapeutic agent from a non-toxic biodegradable polymer system that does not have to be removed from the body after exhaustion of the ophthalmic therapeutic agent from the ophthalmic dispensing device.

These and other objects of the invention will become apparent from a review of the present specification.

### DESCRIPTION OF THE SEVERAL VIEWS OF THE DRAWINGS

Fig. 1 is a photomicrograph of uncompressed microparticles according to Example 1.
Fig. 2 is a photomicrograph of hypercompressed microparticles according to Example 1.
Fig. 3 is a table that reports the level of dexamethasone detected in the vitreous humor and in the aqueous humor.
Fig. 4 is a graph which shows the rate of in vitro release of dexamethasone from microspheres of the invention.

### DETAILED DESCRIPTION OF THE INVENTION

The ophthalmic dispensing device of the invention comprises a polymer that is combined with an ophthalmic therapeutic agent and compressed to form a controlled release dispensing unit. The therapeutic agents that may be mixed with the polymer comprise steroids, non-steroidal anti-inflammatory drugs, antihistamines, antibiotics, mydriatics, beta-adrenergic antagonists, anesthetics, alpha-2-beta adrenergic agonists, mast cell stabilizers, prostaglandin analogues, sympathomimetics, parasympathomimetics, antiproliferative agents, agents to reduce ocular angiogenesis and neovascularization, vasoconstrictors and combinations thereof and any other agents designed to treat eye disease. These agents include but are not limited to particular steroids but include steroids such as prednisone, methylprednisolone, dexamethasone; antibiotics including neomycin, tobramycin, aminoglycosides, fluoroquinolones, polymyxin, sulfacetamide, agents such as pilocarpine, isopilocarpine, physostigmine, demecarium, ecothiphate and acetyl choline and salts thereof; mydriatics and cycloplegics including agents such as atropine, phenylephrine, hydroxyamphetamine, cyclopentolate, homatropine, scopolamine, tropicamide and salts thereof; anesthetics include, lidocaine, proparacaine, tetracaine, phenacaine, and the like;
beta-blockers such as timolol, carteolol, betaxolol, nadolol, levobunolol, carbonic anhydrase inhibitors such as dorzolamide, acetozolamide, prostaglandin analogues such as latanoprost, unoprostone, bimatoprost or travoprost.

The present invention provides an ophthalmic dispensing device that is particularly adapted to the long-term treatment of chronic ocular inflammation and provides an alternative to the use of intravitreal/juxtascleral steroid injections. Chronic ocular inflammation is a frequent cause of opacification of otherwise clear ocular media which may lead to vision loses.

The polymer that is used in combination with the therapeutic agent is a pharmaceutically acceptable polymer that is non-toxic and non-irritating to the eye. These polymers include monomeric and co-polymeric materials. The preferred polymers comprise a biocompatible and biodegradable polymer that may be formed into microparticles known as microspheres or microcapsules which are typically in the size range of about 0.1 to about 150 microns, preferably from about 5 to about 120 and more preferably from about 5 to about 50 microns in diameter. The term microsphere is used to describe a substantially homogeneous structure that is obtained by mixing an active drug with suitable solvents and polymers so that the finished product comprises a drug dispersed evenly in a polymer matrix which is shaped as a microsphere. Depending on the selected size range of the microparticles, the term nanoparticle may be used to describe microspheres having a diameter of between 1 µm to 1 mm. Generally a particle size should be selected so that the particles may be easily measured and transferred as necessary for the purpose of placing the particle in a suitable compression device for the application of pressure to form the hypercompressed dosage form. For this purpose, a preferred range of particle size is from 5 to 50 µm. The hypercompressed particles are designated as the matrix which, when placed in water or in contact with aqueous body fluids, will cause the hypercompressed particles to disaggregate and form into the separate particles that were compressed to form the matrix.
Nanoparticles may be formed for example by sonicating a solution of polylactide polymer in chloroform containing a 2%w/w solution of polyvinyl alcohol in the presence of an ophthalmic therapeutic agent for 10 minutes using a ultasonicator (Misonix XL-2020) at 50-55W power output. Thereafter, the emulsion is stirred overnight at 4°C to evaporate the chloroform and obtain nanoparticles of the polymer and the ophthalmic therapeutic agent.

In general, the microparticles are comprised of from about 0.00001 to about 50
parts by weight of therapeutic agent and is further comprised of from about 50
to about 99.99999 parts by weight of polymer per 100 parts by weight of the total weight of therapeutic agent and polymer. The preferred ranges are from 1 to
50, 5 to 40, and 20 to 30 parts by weight of therapeutic agent, the balance comprised of polymer. If desired from 1 to5wt % of a binder such polyvinyl pyrrolidone may be homogeneously mixed with the microparticles prior to the compression step.

Microspheres may be formed by a typical in-emulsion-solvent-evaporation technique as described herein.

In order to provide a biodegradable polymeric matrix for a controlled release dosage form which is suitable for placement in a position where an ophthalmic therapeutic agent may be released for treatment of a pathology in the eye, it is preferable to select the polymer from poly(alpha hydroxy butyric acid), poly(p-dioxanone) poly(l-lactide), poly(dl-lactide), polyglycolide, poly(glycolide-co-lactide), poly(glycolide-co-dl-lactide), a block polymer of polyglycolide, trimethylene carbonate and polyethylene oxide, or a mixture of any of the foregoing. The lactide/glycolide polymers are bulk-eroding polymers (not surface eroding polymers) and the polymer will hydrolyze when formed into a microparticle matrix as water enters the matrix and the polymer decreases in molecular weight. It is possible to shift the resorption curves to longer times by increasing the polymer molecular weight, using L-polymers and decreasing the surface area by increasing the size of the microparticles or the size of the dosage form. The lactide/glycolide copolymers are available with inherent viscosities as high as 6.5 dl/g and as low as 0.15dl/g. The lower molecular weight copolymers are preferred for the present invention. It has been found that a mol ratio of 50:50 of glycolide to lactide results in the most rapid degradation and the corresponding release of drug. By increasing the ratio of lactide in the polymer backbone from about 50mole % to 100% the rate of release can be reduced to provide an extended therapeutic effect from a single dosage unit.

A preferred encapsulating polymer is poly(glycolide-co-dl-lactide), which serves as the preferred controlled release delivery system for the ophthalmic dispensing device. This device is similar in structure to absorbable polyglycolic acid and polyglycolic/polylactic acid suture materials. The polymeric carrier serves as a sustained-release delivery system for the therapeutic agents. The polymers undergo biodegradation through a process whereby their ester bonds are hydrolyzed to form normal metabolic compounds, lactic acid and glycolic acid and allow for release of the therapeutic agent.

Copolymers consisting of various ratios of lactic and glycolic acids have been studied for differences in rates of degradation. It is known that the biodegradation rate depends on the ratio of lactic acid to glycolic acid in the copolymer, and the 50:50 copolymer degrades most rapidly. The selection of a biodegradable polymer system avoids the necessity of removing an exhausted non-biodegradable structure from the eye with the accompanying trauma.

After the microspheres are prepared, they are compressed to form the ophthalmic dispensing device of the invention. The compression may be carried out in any suitable apparatus that permits the application of 12,000 to 200,000 psi of pressure to the microparticles, and more preferably from 25,000 to 100,000psi, to form the hypercompressed delivery system. The hypercompressed dispensing device may be in the form of a flat disc, a rod shape, or a shaped pellet with rounded or smooth edges, small enough to be placed into the conjunctival fornix, subconjunctival, sub-Tenon's, episcleral, intrascleral, parabulblar, retrobulbar, or intraocular spaces. It is contemplated that the insertion of the ophthalmic dispensing device according to the invention will be carried out by a trained professional as it is contemplated that the method of insertion may involve some manipulation of the eye, using procedures well known to a trained professional in order that the device will be properly placed, Generally, the thickness of the ophthalmic dispensing device should be from about 0.25 to 2mm whether in the form of a disc, rod or pellet. The ophthalmic dispensing device in the form of e.g. a disk, should have an area equal to a circle having a diameter of about 3 to 10mm although smaller or larger devices may be made according to the invention. A rod or cylinder shaped dosage form may be sized to be approximately 1 mm in diameter by 3 mm in length. The density of the ophthalmic dispensing device increases as the amount of compression force is increased. The density should be sufficiently high that it reduces the rate of release of a hypercompressed device that is made using pressures of 12,000 to 200,000psi (or 12Kpsi to 200Kpsi)as compared to uncompressed microparticles.. The hypercompression step also allows for increased drug concentration by consolidating more particles into a finite volume thereby increasing drug loading.. The invention also includes dispensing devices which have two or more drugs formed into microparticles or nanoparticles with a polymer in order to provide controlled release of drugs intended for combination therapy.

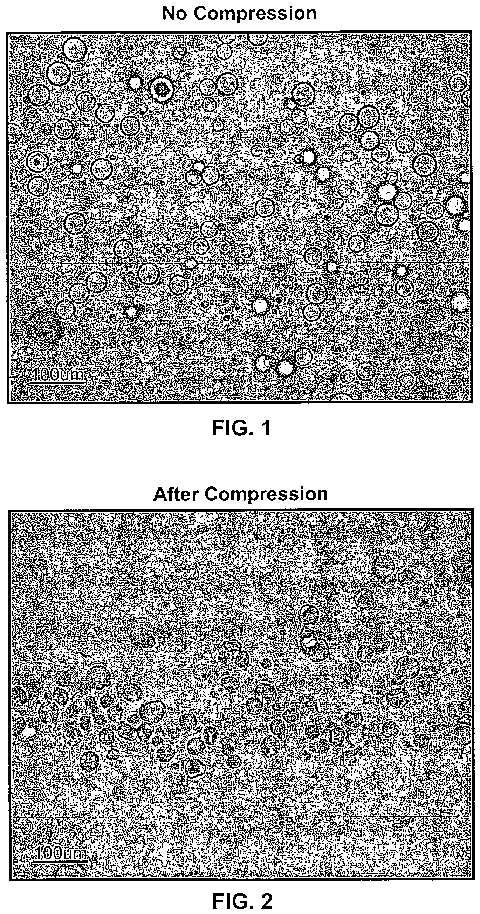

Fig. 1 is a photomicrograph of the microparticles of Example 1 before compression. Fig. 2 is a photomicrograph which shows the physical appearance of hypercompressed microparticles prepared according to the Example 1.
These photomicrographs show the distinct physical change that is effected by the hypercompression of the microcapsules.

### EXAMPLE 1

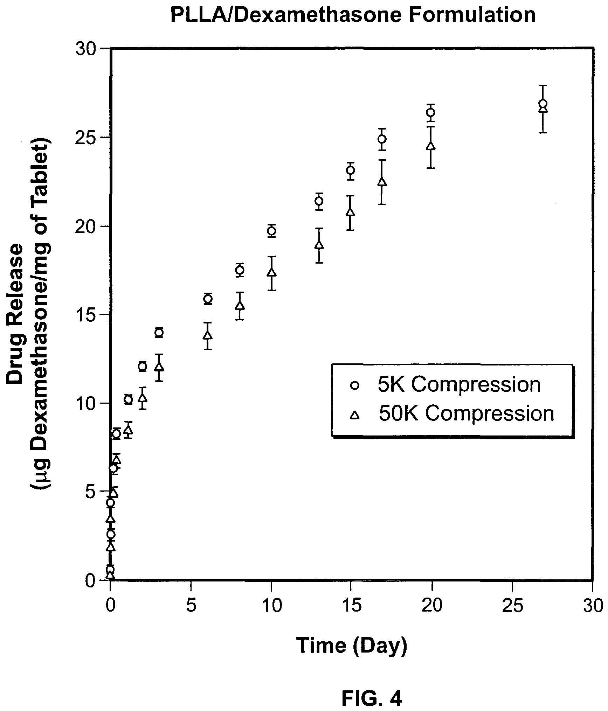

A dosage formulation of dexamethasone, as a hypercompressed microcapsule formulation, is prepared by dispersing 325 mg of dexamethasone in 5g of a poly(dl-lactide) polymer (PLA, intrinsic viscosity 0.66-0.80dl/g as measured in a Ubbelohde viscometer by assessing the flow time of polymer solutions) PLA is dissolved in 125 ml of chloroform and 3.5 ml of ethanol. The suspension is agitated between 1500 to 2000 RPM with 700 ml of a 2% polyvinyl alcohol (30K to 70K MW) maintained at 4° C. After 6 hours of stirring, the agitating speed is reduced to 700 RPM and chloroform is allowed to evaporate over night. The microspheres formed are recovered by centrifugation at 1500 RPM, washed 3 times with water and lyophilized. The microspheres form a free flowing powder having 6.5wt% of dexamethasone with the microspheres having a general diameter in the range of 5 to 25 microns. Thereafter, 250 mg of the microspheres are placed in 7mm diameter stainless steel mold (used for conventional tablet preparation in the pharmaceutical industry) in a MTS mechanical tester modified for compression. A compression force of 5K psi is used to form a first dispensing device and a pressure of 50K psi is used to form a second dispensing device using 60mg of microspheres. The thickness of pellets formed by applying 5K psi of compression pressure is approximately 5.8 mm with a density of 1.06, whereas the thickness for the pellet prepared by applying 50K psi of pressure is approximately 4.2 mm with a density of about 1.55. The dosage form prepared by 50K psi contained 40% more material (by weight) than the dosage form prepared with 5K psi. The dispensing devices prepared using 5K psi and 50K psi were both placed in water. The disc made with 5K psi rapidly disintegrated and dispersed. When the disc made with 5K psi and the disc made with 50K psi were placed in pH 7.4 phosphate buffer, both discs rapidly disintegrated. The in vitro release of dexamethasone from both the 5K psi and 50K psi discs was measured over a 24 hour period of time by placing each disc in a container and filled with pH 7.4 PBS. The containers were placed on an orbital shaker (at ambient temperature) rotating at 100 RPM. At pre-determined time-points, samples were withdrawn and the containers were replenished with fresh aliquots of PBS and the amount of dexamethasone released was determined and is shown in Fig. 4. The results show that the microspheres provide a moderate "initial burst" release of dexamethasone which becomes a pseudo-first order release after one day. The 50K psi disk resulted in a 20% slower. release than the 5K psi disc during this test.

### EXAMPLE 2

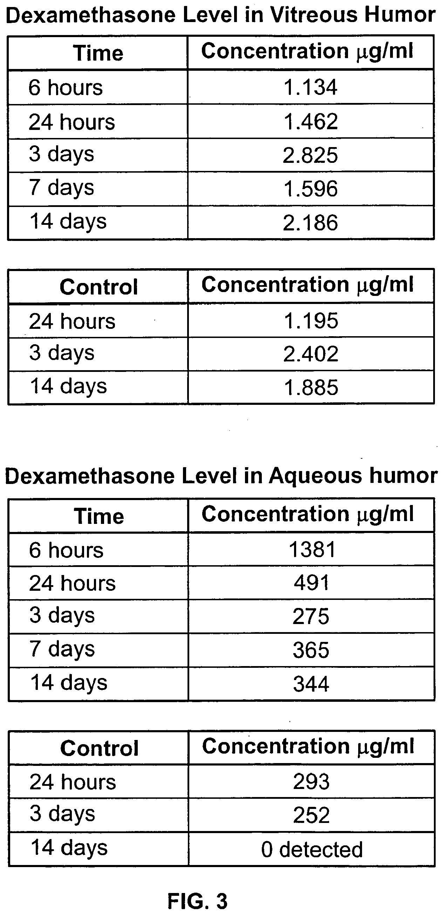

Discs measuring 7 mm in diameter, with a thickness of 1mm, a weight of 60mg, and a drug loading of 6.5%, are made with 50K psi using dexamethasone and the polymer system described above. These discs are placed beneath the conjunctiva in the super temporal quadrant of the eyes of five pigs. The level of dexamethasone in the aqueous humor and the vitreous humor is determined at 0.25 day, 1 day, 3 days, 7 days and 14 days by sampling and analyzing the vitreous humor and the aqueous humor. The concentrations of dexamethasone are reported in Fig. 3. The release profile shown in Fig. 3 shows that the 50K psi disc provided sustained release of dexamethasone for the entire 14 days of the study. Tests of plasma found no detectable dexamethasone which confirmed that the controlled release dosage form has no systemic effect.

## Claims

1. An ophthalmic dispensing device which comprises a polymer which is combined with an ophthalmic therapeutic agent in the form of microparticles which are hypercompressed by the application of 82,737 kPa (12,000 psi) to 1,378,952 kPa (200,000 psi) to form a controlled release dispensing unit, wherein said microparticles are in the form of microspheres obtained by mixing said ophthalmic therapeutic agent with a solvent and a polymer to form a finished product comprising said ophthalmic therapeutic agent dispersed evenly in a polymer matrix shaped as microspheres.

2. An ophthalmic dispensing device as defined in claim 1 where the ophthalmic therapeutic agent is selected from the group consisting of steroids, non-steroidal antiinflammatory drugs, antihistamines, antibiotics, mydriatics, beta-adrenergic antagonists anesthetics, alpha-2-beta adrenergic agonists, mast cell stabilizers, prostaglandin analogues, sympathomimetics, parasympathomimetics, antiproliferative agents, agents to reduce ocular angiogenesis and neovascularization, vasoconstrictors and combinations thereof and any other agents designed to treat eye disease.

3. An ophthalmic dispensing agent as defined in claim 1 where the polymer is selected from the group consisting of poly(alpha hydroxy butyric acid), poly(p-dioxanone) poly(l-lactide), poly(dl-lactide), polyglycolide, poly(glycolide-co-lactide), poly(glycolide-co-dl-lactide), a block polymer of polyglycolide, trimethylene carbonate and polyethylene oxide, or a mixture of any of the foregoing.

4. An ophthalmic dispensing agent as defined in claim 1 where the microparticles have been compressed by the application of 172,369 kPa (25,000 psi) to 689,476 kPa (100,000 psi).

5. An ophthalmic dispensing agent as defined in claim 1 where the therapeutic agent is a steroid.

6. An ophthalmic dispensing agent comprising a polymer in combination with an ophthalmic therapeutic agent in the form of a microparticle, which is hypercompressed by the application of 82,737 kPa (12,000 psi) to 1,378,952 kPa (200,000 psi) to form a controlled release ophthalmic dispensing unit for use in the administration of said ophthalmic drug to the eye, wherein said microparticle is in the form of a microsphere obtained by mixing said ophthalmic therapeutic agent with a solvent and a polymer to form a finished product comprising said ophthalmic therapeutic agent dispersed evenly in a polymer matrix shaped as a microsphere.

7. An ophthalmic dispensing agent as defined in claim 6, where the ophthalmic therapeutic agent is selected from the group consisting of. steroids, non-steroidal anti-inflammatory drugs, antihistamines, antibiotics, mydriatics, beta-adrenergic antagonists, anesthetics, alpha-2-beta adrenergic agonists, mast cell stabilizers, prostaglandin analogues, sympathomimetics, parasympathomimetics, antiproliferative agents, agents to reduce ocular angiogenesis and neovascularization, vasoconstrictors and combinations thereof.

8. An ophthalmic dispensing agent as defined in claim 7, where the polymer is selected from the group consisting of poly(alpha hydroxy butyric acid), poly(p-dioxanone) poly(l-lactide), poly(dl-lactide), polyglycolide, poly(glycolide-co-lactide), poly(glycolide-co-dl-lactide), a block polymer of polyglycolide, trimethylene carbonate and polyethylene oxide, or a mixture of any of the foregoing.

9. An ophthalmic dispensing agent as defined in claim 7, where the polymer and the ophthalmic therapeutic agent are shaped to fit into the eye.

10. ophthalmic dispensing agent as defined in claim 9, where the microparticles have been hypercompressed by the application of 172,369 kPa (25,000 psi) to 344,738 kPa (50,000 psi).

11. An ophthalmic dispensing agent as defined in claim 9, where the ophthalmic therapeutic agent is a steroid.

12. An ophthalmic dispensing agent as defined in claim 10, where the microparticles have been hypercompressed by the application of 344,738 kPa (50,000 psi).

## Patentansprüche

1. Ophtalmische Abgabeeinrichtung, die ein Polymer umfasst, das mit einem ophtalmischen therapeutischen Mittel in Form von Mikroteilchen kombiniert ist, die durch Aufbringung von 82.737 kPa (12.000 psi) bis 1.378.952 kPa (200.000 psi) hyperkomprimiert sind, um eine Abgabeeinheit zur kontrollierten Freisetzung zu bilden, wobei die Mikroteilchen in Form von Mikrokugeln vorliegen, die durch Mischen des ophtalmischen therapeutischen Mittels mit einem Lösungsmittel und einem Polymer erhalten werden, um ein fertiges Produkt zu bilden, umfassend das ophtalmische therapeutische Mittel, das gleichmäßig in einer als Mikrokugeln geformten Polymermatrix dispergiert ist.

2. Ophtalmische Abgabeeinrichtung nach Anspruch 1, wobei das ophtalmische therapeutische Mittel ausgewählt ist aus der Gruppe bestehend aus Steroiden, nichtsteroidalen Antiphlogistika, Antihistaminika, Antibiotika, Mydriatika, beta-adrenergischen Antagonisten, Anästhetika, alpha-2-beta-adrenergischen Agonisten, Mastzellstabilisatoren, Prostaglandin-Analoga, Sympathomimetika, Parasympathomimetika, antiproliferative Mittel, Mittel zur Verminderung von Augenangiogenese und Revaskularisierung, Vasokonstriktiva und Kombinationen davon und alle anderen Mittel, die entwickelt wurden, um Augenkrankheiten zu behandeln.

3. Ophtalmisches Abgabemittel nach Anspruch 1, wobei das Polymer ausgewählt ist aus der Gruppe bestehend aus Poly(alpha-Hydroxybuttersäure), Poly(p-Dioxanon) Poly(1-Lactid), Poly(dl-Lactid), Polyglycolid, Poly(Glycolid-co-Lactid), Poly(Glycolid-co-dl-Lactid), ein Blockpolymer von Polyglycolid, Trimethylencarbonat und Polyethylenoxid oder ein Gemisch aus jedem der vorgenannten Stoffe.

4. Ophtalmisches Abgabemittel nach Anspruch 1, wobei die Mikroteilchen unter Aufbringung von 172.369 kPa (25.000 psi) bis 689.476 kPa (100.000 psi) komprimiert wurden.

5. Ophtalmisches Abgabemittel nach Anspruch 1, wobei das therapeutische Mittel ein Steroid ist.

6. Ophtalmisches Abgabemittel, umfassend ein Polymer in Kombination mit einem ophtalmischen therapeutischen Mittel in Form von Mikroteilchen, das durch Aufbringung von 82.737 kPa (12.000 psi) bis 1.378.952 kPa (200.000 psi) hyperkomprimiert wird, um eine ophtalmische Abgabeeinheit zur kontrollierten Freisetzung zur Verwendung bei der Verabreichung von ophtalmischen Medikamenten an das Auge zu bilden, wobei das Mikroteilchen in Form einer Mikrokugel vorliegt, die durch Mischen des ophtalmischen therapeutischen Mittels mit einem Lösungsmittel und einem Polymer erhalten wird, um ein fertiges Produkt zu bilden, umfassend das ophtalmische therapeutische Mittel, das gleichmäßig in einer als Mikrokugel geformten Polymermatrix dispergiert ist.

7. Ophtalmisches Abgabemittel nach Anspruch 6, wobei das ophtalmische therapeutische Mittel ausgewählt ist aus der Gruppe bestehend aus Steroiden, nichtsteroidalen Antiphlogistika, Antihistaminika, Antibiotika, Mydriatika, beta-adrenergischen Antagonisten, Anästhetika, alpha-2-beta-adrenergischen Agonisten, Mastzellstabilisatoren, Prostaglandin-Analoga, Sympathomimetika, Parasympathomimetika, antiproliferative Mittel, Mittel zur Verminderung von Augenangiogenese und Revaskularisierung, Vasokonstriktiva und Kombinationen davon.

8. Ophtalmisches Abgabemittel nach Anspruch 7, wobei das Polymer ausgewählt ist aus der Gruppe bestehend aus Poly(alpha-Hydroxybuttersäure), Poly(p-Dioxanon) Poly(1-Lactid), Poly(dl-Lactid), Polyglycolid, Poly(Glycolid-co-Lactid), Poly(Glycolid-co-dl-Lactid), ein Blockpolymer von Polyglycolid, Trimethylencarbonat und Polyethylenoxid oder ein Gemisch aus jedem der vorgenannten Stoffe.

9. Ophtalmisches Abgabemittel nach Anspruch 7, wobei das Polymer und das ophtalmische therapeutische Mittel so geformt sind, dass sie in das Auge passen.

10. Ophtalmisches Abgabemittel nach Anspruch 9, wobei die Mikroteilchen unter Aufbringung von 172.369 kPa (25.000 psi) bis 344.738 kPa (50.000 psi) hyperkomprimiert wurden.

11. Ophtalmisches Abgabemittel nach Anspruch 9, wobei das ophtalmische therapeutische Mittel ein Steroid ist.

12. Ophtalmisches Abgabemittel nach Anspruch 10, wobei die Mikroteilchen unter Aufbringung von 344.738 kPa (50.000 psi) hyperkomprimiert wurden.

## Revendications

1. Dispositif de distribution ophtalmique comprenant un polymère qui est combiné avec un agent thérapeutique ophtalmique sous la forme de microparticules hypercompressées par application de 82 737 kPa (12 000 psi) à 1 378 952 kPa (200 000 psi) pour former une unité de distribution à libération contrôlée, dans lequel lesdites microparticules ont la forme de microsphères obtenues en mélangeant ledit agent thérapeutique ophtalmique avec un solvant et un polymère pour former un produit fini comprenant ledit agent thérapeutique ophtalmique dispersé uniformément dans une matrice polymère ayant la forme de microsphères.

2. Dispositif de distribution ophtalmique selon la revendication 1, dans lequel l'agent thérapeutique ophtalmique est choisi parmi des stéroïdes, des anti-inflammatoires non stéroïdiens, des antihistaminiques, des antibiotiques, des mydriatiques, des anesthésiques bêta-adrénergiques, des agonistes alpha-2-bêta-adrénergiques, des stabilisateurs de mastocytes, des analogues de prostaglandines, des sympathomimétiques, des parasympathomimétiques, des agents antiprolifératifs, des agents réduisant l'angiogenèse oculaire et la néovascularisation, des vasoconstricteurs et des combinaisons de ceux-ci et tout autre agent conçu pour traiter les maladies oculaires.

3. Agent de distribution ophtalmique selon la revendication 1, dans lequel le polymère est choisi parmi l'acide poly(alpha hydroxy butyrique), le poly(p-dioxanone) poly(1-lactide), le poly(dl-lactide), le polyglycolide, le poly(glycolide-co-lactide), poly(glycolide-co-dl-lactide), un polymère en bloc de polyglycolide, le carbonate de triméthylène et l'oxyde de polyéthylène, ou un mélange quelconque de ces derniers.

4. Agent de distribution ophtalmique selon la revendication 1, dans lequel les microparticules ont été comprimées par l'application d'une pression de 172 369 kPa (25 000 psi) à 689 476 kPa (100 000 psi).

5. Agent de distribution ophtalmique selon la revendication 1, dans lequel l'agent thérapeutique est un stéroïde.

6. Agent de distribution ophtalmique comprenant un polymère combiné à un agent thérapeutique ophtalmique sous la forme d'une microparticule, qui est hypercompressée par application d'une pression de 82 737 kPa (12 000 psi) à 1 378 952 kPa (200 000 psi) pour former une unité de distribution ophtalmique à libération contrôlée pour utilisation dans l'administration dudit médicament ophtalmique à l'oeil, dans lequel ladite microparticule est sous la forme d'une microsphère obtenue en mélangeant ledit agent thérapeutique ophtalmique avec un solvant et un polymère pour former un produit fini comprenant ledit agent thérapeutique ophtalmique dispersé uniformément dans un polymère matrice en forme de microsphère.

7. Agent de distribution ophtalmique selon la revendication 6, dans lequel l'agent thérapeutique ophtalmique est choisi parmi des stéroïdes, des anti-inflammatoires non stéroïdiens, des antihistaminiques, des antibiotiques, des mydriatiques, des anesthésiques bêta-adrénergiques, des agonistes alpha-2-bêta-adrénergiques, des stabilisateurs de mastocytes, des analogues de prostaglandines, des sympathomimétiques, des parasympathomimétiques, des agents antiprolifératifs, des agents réduisant l'angiogenèse oculaire et la néovascularisation, des vasoconstricteurs et des combinaisons de ceux-ci.

8. Agent de distribution ophtalmique selon la revendication 7, dans lequel le polymère est choisi parmi l'acide poly(alpha hydroxy butyrique), le poly(p-dioxanone) poly(l-lactide), le poly(dl-lactide), le polyglycolide, le poly(glycolide-co-lactide), poly(glycolide-co-dl-lactide), un polymère en bloc de polyglycolide, le carbonate de triméthylène et l'oxyde de polyéthylène, ou un mélange quelconque de ces derniers.

9. Agent de distribution ophtalmique selon la revendication 7, dans lequel le polymère et l'agent thérapeutique ophtalmique sont conformés pour s'adapter à l'oeil.

10. Agent de distribution ophtalmique selon la revendication 9, dans lequel les microparticules ont été hypercompressées par l'application d'une pression de 172 369 kPa (25 000 psi) à 344 738 kPa (50 000 psi).

11. Agent de distribution ophtalmique selon la revendication 9, dans lequel l'agent thérapeutique ophtalmique est un stéroïde.

12. Agent de distribution ophtalmique selon la revendication 10, dans lequel les microparticules ont été hypercompressées par l'application d'une pression de 344 738 kPa (50 000 psi).
